# EUROPEAN PATENT SPECIFICATION

(11) **EP 1 871 368 B1**
(45) Date of publication and mention of the grant of the patent: **08.06.2011**
(21) Application number: 06740387.3
(22) Date of filing: 04.04.2006
(51) Int. Cl.: A61K 31/445, A61K 31/4412, A61P 25/28

(54) **DIHYDROPYRIDINE COMPOUNDS FOR NEURODEGENERATIVE DISEASES AND DEMENTIA**
DIHYDROPYRIDIN-VERBINDUNGEN FÜR NEURODEGENERATIVE ERKRANKUNGEN UND DEMENZ
COMPOSES DE DIHYDROPYRIDINE UTILISES POUR TRAITER DES MALADIES NEURODEGENERATIVES ET LA DEMENCE

(30) Priority: 04.04.2005 US 667665 P; 13.06.2005 US 689519 P
(43) Date of publication of application: 02.01.2008
(73) Proprietor: Eisai R&D Management Co., Ltd., Bunkyo-ku Tokyo 112-8088 (JP)
(72) Inventor: GRAY, Julian, A., D-79594 Inzlingen (DE); YOSHIHARU, Yamanishi, Tsukuba-shi, Ibaraki, 300-2635 (JP); NOBUYUKI, Mori, Tsukuba-shi, Ibaraki 300-2635 (JP); FIELDS, Scott, Ridgefield Park, New Jersey 07660 (US)
(74) Representative: Woods, Geoffrey Corlett
(86) International application number: PCT/US2006/012284
(87) International publication number: WO 2006/107859

(56) References cited:
- US-A1- 2003 144 255
- US-A1- 2004 023 973
- US-A1- 2006 100 249
- SHIGETA M ET AL: "DONEPEZIL FOR ALZHEIMER'S DISEASE: PHARMACODYNAMIC, PHARMACOKINETIC, AND CLINICAL PROFILES" CNS DRUG REVIEWS, BRANFORD, CT, US, vol. 7, no. 4, 21 December 2001 (2001-12-21), pages 353-368, XP009028267 ISSN: 1080-563X

## Description

The invention relates to a dihydropyridine compounds for use in combination with a cholinesterase inhibitor for treating a variety of diseases and disorders. The invention also relates to a cholinesterase inhibitor for use in combination with a dihydropyridine compound for treating a variety of diseases and disorders.

### Background of the Invention

Excitatory amino acid receptors are classified into two general types. Receptors that are directly coupled to the opening of cation channels in the cell membrane of the neurons are termed "ionotropic." This type of receptor has been subdivided into at least three subtypes, which are defined by the depolarizing actions of the selective agonist N-methyl-D-aspartate (NMDA), α-amino-3-hyoroxy-5-methylisoxazole-4-propionic acid (AMPA), and kainic acid. The second general type is the G-protein or second messenger-linked "metabotropic" excitatory amino acid receptor. This second type, when activated by the agonists quisqualate, ibotenate, or trans-1-aminocyclopentane-1,3-dicarboxylic acid, leads to enhanced phosphoinositide hydrolysis in the postsynaptic cell. Both types of receptors appear not only to mediate normal synaptic connections during development, but also change in the efficiency of synaptic transmission throughout life. Schoepp et al, Trends Pharm Sci., 11:508 (1990); McDonald et al, Brain Res. Rev., 15:41 (1990).

Excessive excitation by neurotransmitters can cause the degeneration and death of neurons. It is believed that this degeneration is in part mediated by the excitotoxic actions of the excitatory ammo acids, glutamate and aspartate, at the NMDA receptor, the AMPA receptor, and the kainate receptor. This excitotoxic action is responsible for the loss of neurons in cerebrovascular disorders such as cerebral ischemia or cerebral infarction resulting from a range of conditions, such as thromboembolic or haemorrhagic stroke, cerebral vasospasm, hypoglycaemia, cardiac arrest, status epilepticus, perinatal asphyxia, anoxia such as from near-drowning, pulmonary surgery and cerebral trauma as well as lathyrism, Alzheimer's, and Huntingdon's diseases. U.S. Patent Nos. 6,765,006 and 5,843,945

US 2004/0023973 describes 1-2-dihydropyridine compounds and their use as inhibitors to non-NMDA receptors, particularly an AMPA receptor.

Shigeta et al. (CNS Drug Reviews, Branford, CT, US, vol. 7, no.4, 21 December 2001, 353-368) describes the use of donepezil for treating Alzheimer's disease and describes donepezil's pharmacodynamic, pharmacokinetic and clinical profiles.

US 2006/0100249 describes compositions comprising 1,2-dihydropyridin-2-one compounds and an immunoregulatory or anti-inflammatory agent, and their use in treating neurodegenerative diseases.

US 2003/0144255 describes a pharmaceutical composition comprising a 4,4'-diaminodiphenylsulphone compound in combination with a compound having cholinesterase inhibiting activity, and their use in treating dementia.

There is a need in the art for new compounds and new methods of treating diseases and disorders that may be mediated to any extent by NMDA receptors, AMPA receptors and/or kainate receptors. The invention is directed to this, as well as other, important ends.

### Summary of the Invention

The invention provides a compound for use in combination with donepezil or a pharmaceutically acceptable salt thereof in the treatment of (i) Alzheimer's disease in a patient or (ii) dementia or one or more cognitive impairments in a patient; wherein the compound is 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof.

The present invention also provides a compound for use in combination with 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof in the treatment of (i) Alzheimer's disease in a patient or (ii) dementia or one or more cognitive impairments in a patient; wherein the compound is donepezil or a pharmaceutically acceptable salt thereof.

The present invention also provides use of 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof in the manufacture of a medicament for use in combination with donepezil or a pharmaceutically acceptable salt thereof in the treatment of (i) Alzheimer's disease in a patient or (ii) dementia or one or more cognitive impairments in a patient.

The present invention also provides use of donepezil or a pharmaceutically acceptable salt thereof in the manufacture of a medicament for use in combination with 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof in the treatment of (i) Alzheimer's disease in a patient or (ii) dementia or one or more cognitive impairments in a patient.

The donepezil or the pharmaceutically acceptable salt thereof and 3-(2-cyanophenyl)-5-(2-pyridyl)-1-phenyl-1,2-dihydropyridine, the pharmaceutically acceptable salt thereof, the hydrate thereof or the hydrate of a pharmaceutically acceptable salt thereof for use in the present invention may be (a) administered separately to the patient or (b) administered to the patient in the form of a pharmaceutical composition.

The dementia may be caused by Alzheimer's disease, Parkinson's disease, or Huntington's disease.

The combination of the 1,2-dihydropyridine compound and donepezil unexpectedly produces synergistic effects in the treatment and/or prophylaxis of dementia or cognitive impairments.

The combination of the 1,2-dihydropyridine compound and donepezil also unexpectedly produce synergistic effects in the treatment of neurodegenerative diseases.

### Brief Description of the Figures

Figure 1 shows the effect of Compound A (i.e., 3-(2-cyanophenyl)-5-(2-pyridyl)-1-phenyl-1,2-dihydmpyridin-2-one) on dopamine, DOPAC, and HVA content in mice striatum.
Figure 2 shows the effects of donepezil on dopamine turnover in mice striatum.
Figure 3A-E show the effects of a combination of Compound A (i.e., 3-(2-cyanophenyl)-5-(2-pyridyl)-1-phenyl-1,2-dihydropyridin-2-one) and donepezil on dopamine turnover, DOPAC, HVA, DOPAC/DA and HVA/DA, respectively, in mice striatum.
Figure 4 shows the effects of Compound A (i.e., 3-(2-cyanophenyl)-5-(2-pyridyl)-1-phenyl-1,2-dihydropyridin-2-one) and the combination of Compound A (i.e., 3-(2-cyanophenyl)-5-(2-pyridyl)-1-phenyl-1,2-dihydropyridin-2-one) and donepezil on kainate-induced neuronal toxicity in cortical neurons. The * indicates that p<0.05 vs. control, and # indicates that p<0.05 vs. donepezil alone.
Figure 5 shows the effect of Compound A (i.e., 3-(2-cyanophenyl)-5-(2-pyridyl)-1-phenyl-1,2-dihydropyridin-2-one) and the combination treatment of Compound A (i.e., 3-(2-cyanophenyl)-5-(2-pyridyl)-1-phenyl-1,2-dihydropyridin-2-one) and donepezil on NMDA-induced neural toxicity in cortical neurons. The * indicates that p<0.05 vs. control.

### Detailed Description of the Invention

"Patient" refers to animals, preferably mammals, more preferably humans. The term "patient" includes men and women; and includes adults, children and neonates. In one embodiment, the patient can be an animal companion, such as a dog or a cat.

"Cognitive impairments" refer to an acquired deficit in one or more of memory function, problem solving, orientation, and abstraction that impinges on a patient's ability to function independently.

"Dementia" refers to a global deterioration of intellectual functioning in clear consciousness, and is characterized by one or more symptoms of disorientation, impaired memory, impaired judgment, and impaired intellect.

"Administered separately" with reference to the administration of two or more compounds to treat and/or prevent the diseases and disorders described herein includes, for example, the sequential administration of the compounds in any order or the simultaneous administration of the compounds. Simultaneous administration of the compounds means that the compounds are administered to the patient at substantially the same time or at exactly the same time, depending on the mode of administration. The sequential administration of the compounds may occur in any order and may occur with any amount of time elapsing between administration of the compounds. Sequential administration may be based on factors that would influence which of the compounds should be administered first and which should be administered second, and how much time should elapse between administration of the compounds. For example, when two or more compounds are administered separately and sequentially, factors that effect when the compounds are administered to the patient include, for example, (a) the time(s) that provides the best efficacy for the compound being administered, (b) the time(s) that provides the fewest side effects for the compound being administered, (c) the dosage of the compound, (d) the route of administration of the compound, (e) the disease or disorder being treated, (f) the patient being treated, (g) the *in vivo* relationship of the compounds being administered, and other such factors known in the art. Preferably, the time intervals for sequential administration are chosen so that the effect on the disease or disorder being treated in the combined use of the active ingredients is greater than additive when compared to the effect which would be obtained by use of only one of the active ingredients.

The term "combination" refers to the 1,2-dihydropyridine compound and the second active ingredient (donepezil or a pharmaceutically acceptable salt thereof) being administered separately as distinct pharmaceutical compositions or formulations. The pharmaceutical compositions or formulations can have the same or different modes of administration.

"Active ingredient" refers to the 1,2-dihydropyridine compound described herein and donepezil of a pharmaceutically acceptable salt thereof described herein that are responsible for treatment and/or prophylaxis of a disease or disorder.

"Monotherapy" is a therapy which uses only one active ingredient for treatment and/or prophylaxis of a disease or disorder.

"Combination therapy" is a therapy where two or more active ingredients are administered separately or are administered in the form of a pharmaceutical composition for the treatment and/or prophylaxis of a disease.

"Therapeutically effective amount" refers to the amount of the active ingredient that is necessary for the treatment and/or prophylaxis of a disease. When two or more active ingredients are administered for combination therapy, the term "therapeutically effective amount" refers to the amount of active ingredients that are necessary for treatment and/or prophylaxis of a disease and includes, for example: (a) a therapeutically effective amount of a first active ingredient and a therapeutically effective amount of a second active ingredient (i.e., the amount of each active ingredient that would be used for monotherapy for the treatment and/or prophylaxis of a disease is used for the combination therapy); (b) a therapeutically effective amount of a first active ingredient and a sub-therapeutic amount of a second active ingredient, which in combination effectively provide for treatment and/or prophylaxis of a disease (e.g., the sub-therapeutic amount of the second active ingredient can be used in combination therapy to achieve a result that would be equal to or greater than the result that the second active ingredient would achieve if it was used for monotherapy); (b) a sub-therapeutic amount of a first active ingredient and a therapeutically effective amount of a second active ingredient, which in combination effectively provide for treatment and/or prophylaxis of a disease (e.g., the sub-therapeutic amount of the first active ingredient can be used in combination therapy to achieve a result that would be equal to or greater than the result that the first active ingredient would achieve if it was used for monotherapy); and (d) a sub-therapeutic amount of a first active ingredient and a sub-therapeutic amount of a second active ingredient, which in combination therapy provide for treatment and/or prophylaxis of a disease or disorder (e.g., the subtherapeutic amount of the first active ingredient can be used in combination therapy to achieve a result that would be equal to or greater than the result that the first active ingredient would achieve if it was used for monotherapy; and the sub-therapeutic amount of the second active ingredient can be used in combination therapy to achieve a result that would be equal to or greater than the result that the second active ingredient would achieve if it was used for monotherapy).

"Commercial packages," also known as kits, can include a combination of (i) a first pharmaceutical composition or formulation comprising the 1,2-dihydropyridine compound); (ii) a second pharmaceutical composition or formulation comprising the second active ingredient (donepezil or a pharmaceutically acceptable salt thereof); (iii) instructions approved by the FDA for using the pharmaceutical compositions or formulations for treating or preventing the disease; and (iv) optionally other materials to administer the pharmaceutical compositions or formulations (e.g., syringes, diluents, medical gloves, hand sanitizers, and the like); to monitor drug levels in the body; to support patient compliance with medication dosing; or to monitor the status of the disease. The commercial package can supply enough medication and materials for days, weeks or months. Also described herein are "commercial packages" which can include (i) pharmaceutical composition or formulation comprising both the 1,2-dihydropyridine compound and the second active ingredient (donepezil or a pharmaceutically acceptable salt thereof); (ii) instructions approved by the FDA for using the pharmaceutical composition or formulation for treating or preventing the disease; and (ill) optionally other materials to administer the pharmaceutical compositions or formulations (e.g., syringes, diluents, medical gloves, hand sanitizers, and the like); to monitor drug levels in the body; to support patient compliance with medication dosing, or to monitor the status of the disease. The commercial package can supply enough medication and materials for days, weeks or months.

"Hydrate" refers to a compound containing a molecule of water of crystallization. The molecule of water of crystallization can be an integer of 1 or more, such as 1 to 10; or can be any fraction greater than 0 or a fraction of an integer from 1 to 10. For example, the hydrate may be represented as compound·¼H₂O; compound·½H₂O; compound·¾H₂O; compound·2H₂O; compound·5½H₂O; compound·6H₂O; and the like. The "compound" can be any described herein, such as 3-(2-cyanophenyl)-5-(2-pyridyl)-1-phenyl-1,2-dihydropyridin-2-one.

"Pharmaceutically acceptable salts" are well known in the art and include those of inorganic acids, such as hydrochloride, sulfate, hydrobromide and phosphate; and those of organic acids, such as formate, acetate, trifluoroacetate, methanesulfonate, benzenesulfonate and toluenesulfonate. When certain substituents are selected, the compounds of the invention can form, for example, alkali metal salts, such as sodium or potassium salts; alkaline earth metal salts, such as calcium or magnesium salts; organic amine salts, such as a salt with trimethyl-amine, triethylamine, pyridine, picoline, dicyclohexylamine or N,N'-dibenzylethylenediamine. One skilled in the art will recognize that the compounds of the invention can be made in the form of any other pharmaceutically acceptable salt

The term "1,2-dihydropyridine compound" used herein includes the 1,2-dihydropyridine compounds, pharmaceutically acceptable salts of the 1,2-dihydropyridine compound, stereoisomers of the 1,2-dihydropyridine compound, pharmaceutically acceptable salts of stereoisomers of the 1,2-dihydropyridine compound, hydrates of the 1,2-dihydropyridine compounds, hydrates of pharmarceutically acceptable salts of the 1,2-dihydropyridine compound, stereoisomers of hydrates of the 1,2-dihydropyridine compound, and stereoisomer of hydrates of pharmaceutically acceptable salts of the 1,2-dihydropyridine compound.

The 1,2-dihydropyridine compound used in the methods and compositions described herein is Compound A: 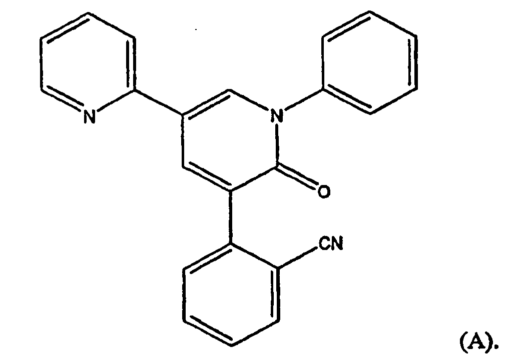 The IUPAC name for Compound A is 2-(2-oxo-1-phenyl-5-pyridin-2-yl-1,2-dihydropyridin-3-yl)benzonitrile. Compound A may also be referred to as 3-(2-cyanophenyl)-5-(2-pyridyl)-1-phenyl-1,2-dihydropyndin-2-one.

Throughout the specification, the terms "Compound A," "2-(2-oxo-1-phenyl-5-pyridin-2-yl-1,2-dihydropyridin-3-yl)benzonitrile," and "3-(2-cyanophenyl)-5-(2-pyndyl)-1-phenyl-1,2-dihydropyridin-2-one" are intended to include pharmaceutically acceptable salts thereof, stereoisomers thereof, pharmaceutically acceptable salts of stereoisomers thereof, hydrates thereof, hydrates of pharmaceutically acceptable salts thereof, stereoisomers of hydrates thereof, and stereoisomer of hydrates of pharmaceutically acceptable salts thereof. In another embodiment, the terms "Compound A," "2-(2-oxo-1-phenyl-5-pyridin-2-yl-1,2-dihydropyridin-3-yl)benzonitrile," and "3-(2-cyanophenyl)-5-(2-pyridyl)-1-phenyl-1,2-dihydropyridin-2-one" are intended to include pharmaceutically acceptable salts thereof, hydrates thereof, and hydrates of pharmaceutically acceptable salts thereof.

The 1,2-dihydropyndine compound and methods for making the 1,2-dihydropyridine compound used in the present invention are described in US Patent No. 6,949,571, US Publication No. 2004/0023973, and PCT Publication No. WO 03/047577, WO 04/009553, WO 06/004100, and WO 06/004107, the disclosures of which are incorporated by reference herein in their entirety. Methods for making other AMPA receptor antagonists are described in WO 2005/094797.

The present invention preferably employs 1-benzyl-4-((5,6-dimethoxy-1-indanon)-2-yl)methylpiperidine; which is represented by formula (B):

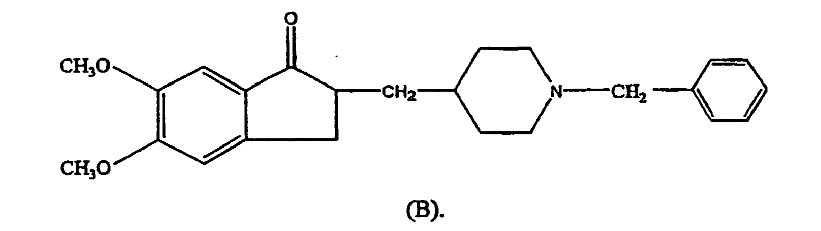

The compound of Formula (B), known as donepezil, may be in the form of a polymorph or a polymorphic crystal. For example, donepezil may be in the form of polymorph (II), (III), (IV), or (V); preferably polymorph (III). Donepezil may be in the form of polymorphic crystals (A), (B), or (C). Polymorphs, polymorphic crystals, and methods for making polymorphs and polymorphic crystals are described in U.S. Patent Nos. 5,985,864, 6,140,321 and 6,245,911, the disclosures of which are incorporated by reference herein in their entirety.

In another embodiment, the present invention employs 1-benzyl-4-((5,6-dimethoxy-1-indanon)-2-yl)methylpiperidine hydrochloride, which is also known as donepezil hydrochloride, and which is represented by formula (B1):

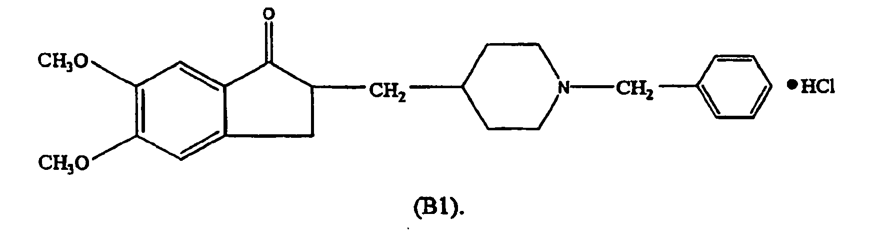

The compounds of the invention can have an asymmetric carbon atom(s), depending upon the substituents, and can have stereoisomers, which are within the scope of the invention. For example, donepezil or pharmaceutically acceptable salts thereof can be in the forms described in Japanese Patent Application Nos. 4-187674 and 4-21670.

Japanese Patent Application No. 4-187674 describes a compound of formula (B2): 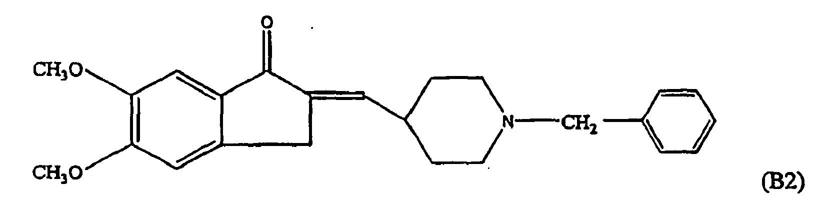 which can be in the form of a pharmaceutically acceptable salt, such as a hydrochloride salt. Japanese Patent Application No. 4-21670 describes compounds of formula (B3): 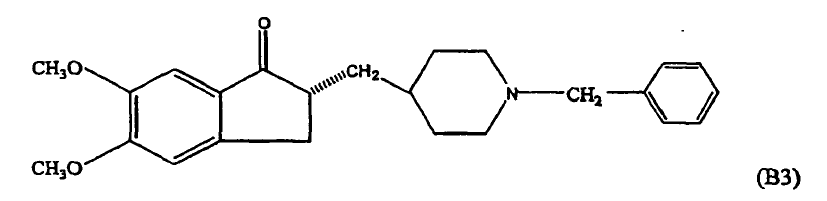 which can be in the form of a pharmaceutically acceptable salt, such as a hydrochloride salt; and compounds of formula (B4): 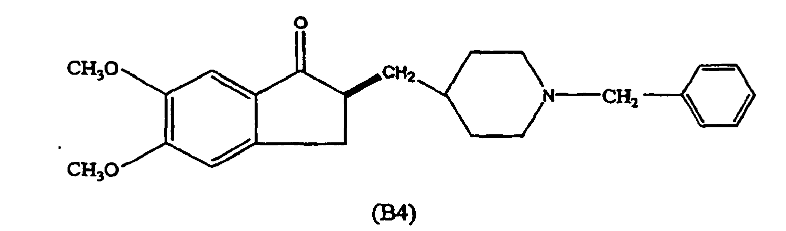 which can be in the form of a pharmaceutically acceptable salt, such as a hydrochloride salt; and compounds of formula (B5):

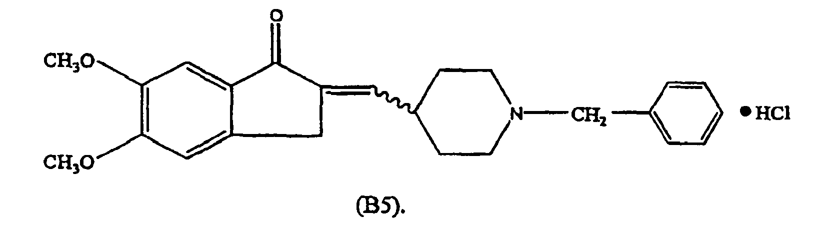

Throughout the specification, the terms "donepezil" and "1 -benzyl-4-((5,6-dimethoxy-1-indanon)-2-yl)methylpiperidine" are intended to include one or more of the following (e.g., combinations of two or more thereof): pharmaceutically acceptable salts; stereoisomers; polymorphs and polymorphic crystals.

The donepezil described herein is commercially available or can be prepared by processes known in the art, such as those described, for example, in U.S. Patent Nos. 4,895,841, 5,985,864, 6,140,321 and 6,245,911; WO 98/39000, and Japanese Patent Application Nos. 4-187674 and 4-21670.

Also described herein are pharmaceutical compositions comprising a therapeutically effective amount of: (i) at least one AMPA receptor antagonist; (ii) at least one nootropic; and (iii) a pharmaceutically acceptable excipient. The AMPA receptor antagonist is the 1,2-dihydropyridine compound described herein. The nootropic is the cholinesterase inhibitor donepezil or a pharmaceutically acceptable salt thereof described herein.

Also described herein use combinations comprising a therapeutically effective amount of (i) at least one AMPA receptor antagonist and (ii) at least one nootropic. The combination may be administered separately (e.g., simultaneously, sequentially) to a patient to treat the diseases or disorders described herein (e.g., cognitive impairments, dementia and the like). The AMPA receptor antagonist is the 1,2-dihydropyridine compound described herein. The nootropic is the cholinesterase inhibitor donepezil or a pharmaceutically acceptable salt thereof described herein.

Also described herein are commercial packages (e.g., kits) comprising a therapeutically effective amount of: (i) at least one AMPA receptor antagonist and (ii) at least one nootropic; and (iii) instructions for simultaneous, separate or sequential use thereof in the treatment of the diseases and disorders described herein. The AMPA receptor antagonist is the 1,2-dihydropyridine compound described herein. The nootropic is the cholinesterase inhibitor donepezil or a pharmaceutically acceptable salt thereof described herein.

Also described herein are pharmaceutical compositions comprising a therapeutically effective amount of: (i) the 1,2-dihydropyridine compound described herein (ii) donepezil or a pharmaceutically acceptable salt thereof inhibitor, and (iii) at least one pharmaceutically acceptable excipient. Further described herein are combinations comprising a therapeutically effective amount of: (i) the 1,2-dihydropyridine compound described herein and (ii) donepezil or a pharmaceutically acceptable salt thereof; wherein the compounds may be administered separately (e.g., simultaneously, sequentially) to a patient to treat the diseases or disorders described. Also described herein are commercial packages (e.g., kits) comprising a therapeutically effective amount of: (i) the 1,2-dihydropyridine compound described herein (ii) donepezil or a pharmaceutically acceptable salt thereof; and (iii) instructions for the simultaneous, separate or sequential use of (i) and (ii) in the treatment of the diseases and disorders described herein. The 1,2-dihydropyridine compound can be any described herein. The pharmaceutical compositions may comprise a therapeutically effective amount of: (i) donepezil; (ii) 3-(2-cyanophenyl)-5-(2-pyridyl)-1-phenyl-1,2-dihydropyridin-2-one; and (iii) at least one pharmaceutically acceptable excipient.

Further described herein are (i) methods for treating one or more cognitive impairments in a patient in need thereof; (ii) methods for treating dementia in a patient in need thereof; and (iii) methods for delaying the onset of dementia or one more cognitive impairments in a patient in need thereof by administering a therapeutically effective amount of: (a) the 1,2-dihydropyridine compound described herein and (b) donepezil or a pharmaceutically acceptable salt thereof. The 1,2-dihydropyridine compound and the donepezil or a pharmaceutically acceptable salt thereof can be administered separately or can be administered in the form of a composition.

The methods for treating dementia include methods for treating the behavioral disturbances associated with dementia. Exemplary behavioral disturbances include sexual disinhibition, changes in activity, changes in interpersonal relationships, physical aggressiveness, physical non-aggressiveness (e.g., wandering), verbal aggressiveness, and verbal non-aggressiveness (e.g., repetitive vocalization).

The cause(s) of the cognitive impairment(s) or dementia may be known or unknown. For example, dementia and cognitive impairment(s) may be caused by Alzheimer's disease, Parkinson's disease, Huntington's disease, Pick's disease, Lewy body disease, vascular disease (e.g., cerebrovascular disease), HIV, AIDS, epilepsy, brain tumors, brain lesions, multiple sclerosis, Down's syndrome, Rett's syndrome, progressive supranuclear palsy, frontal lobe syndrome, schizophrenia, traumatic brain injuries (e.g., closed head injuries), post coronary artery by-pass graft surgery, electroconvulsive shock therapy, chemotherapy, radiation therapy, radiation exposure, encephalitis, meningitis, fetal alcohol syndrome, Korsakoffs syndrome, anoxic brain injury, cardiopulmonary resuscitation, diabetes, menopause, strokes, high cholesterol levels, or spinal cord disorders (e.g., spinal cord injury, spinal cord ischemia, spinal cord infarction, spinal cord convulsions). In one embodiment, the cognitive impairments may be mild cognitive impairments or age-associated cognitive impairments. For additional descriptions of cognitive impairments, dementia, and the causes of cognitive impairments and dementia, the disclosures of US Patent No. 6,458,807, US Publication No. 2006/0018839, and WO 2005/074535 are incorporated by reference herein in their entirety.

The cognitive impairments may be canine cognitive impairments or dysfunctions (CCD), which are the age related deterioration of cognitive abilities in dogs characterized by behavioral changes that cannot be wholly attributed to general medical conditions. When preparing veterinary compositions, one skilled in the art would appreciate that the composition would contain a therapeutically effective amount of: (a) the 1,2-dihydropyridine compound described herein (e.g., 3-(2-cyanophenyl)-5-(2-pyridyl)-1-phenyl-1,2-dihydropyridin-2-one), (b) donepezil or a pharmaceutically acceptable salt thereof, and (c) a veterinarily acceptable carrier. The dosage amount for a dog can be easily achieved based on the dosages that are acceptable for humans and the size and weight of the dog.

Also described herein are (i) methods for treating a neurodegenerative disease; and (ii) methods for delaying the onset of a neurodegenerative disease in a patient in need thereof by administering a therapeutically effective amount of: (a) the 1,2-dihydropyridine compound described herein and (b) donepezil or a pharmaceutically acceptable salt thereof. The 1,2-dihydropyridine compound and the donepezil or a pharmaceutically acceptable salt thereof can be administered separately or can be administered in the form of a composition. The neurodegenerative disease may be any known in the art Exemplary neurodegenerative diseases include Alzheimer's disease, Parkinson's disease, Huntington's disease, amyotrophic lateral sclerosis, Picks disease, Lewy body disease, prion diseases (e.g., Creutzfeldt-Jakob disease), multiple sclerosis, epilepsy, strokes, and the like.

Also described herein are (i) methods for treating Alzheimer's disease; and (ii) methods for delaying the onset of Alzheimer's disease in a patient in need thereof by administering a therapeutically effective amount of: (a) the 1,2-dihydropyridine compound described herein and (b) donepezil or a pharmaceutically acceptable salt thereof. The Alzheimer's disease may be mild, moderate, or severe. The 1,2-dihydropyridine compound and the donepezil or a pharmaceutically acceptable salt thereof can be administered separately or can be administered in the form of a composition.

Also described herein are (i) methods for treating Parkinson's disease; and (ii) methods for delaying the onset of Parkinson's disease in a patient in need thereof by administering a therapeutically effective amount of: (a) the 1,2-dihydropyridine compound described herein and (b) donepezil or a pharmaceutically acceptable salt thereof. The 1,2-dihydropyridine compound and the donepezil or a pharmaceutically acceptable salt thereof can be administered separately or can be administered in the form of a composition.

Also described herein use (i) methods for treating Huntington's disease; and (ii) methods for delaying the onset of Huntington's disease in a patient in need thereof by administering a therapeutically effective amount of: (a) the 1,2-dihydropyridine compound described herein and (b) donepezil or a pharmaceutically acceptable salt thereof. The 1,2-dihydropyridine compound and the donepezil or a pharmaceutically acceptable salt thereof can be administered separately or can be administered in the form of a composition.

Also described herein are (i) methods for treating amyotrophic lateral sclerosis; and (ii) methods for delaying the onset of amyotrophic lateral sclerosis in a patient in need thereof by administering a therapeutically effective amount of: (a) the 1,2-dihydropyridine compound described herein and (b) donepezil or a pharmaceutically acceptable salt thereof. The 1,2-dihydropyridine compound and the donepezil or a pharmaceutically acceptable salt thereof can be administered separately or can be administered in the form of a composition.

The 1,2-dihydropyridine compounds described herein, and the donepezil or a pharmaceutically acceptable salt thereof can be administered orally, topically, parenterally, by inhalation (nasal or oral), or rectally in dosage unit formulations containing conventional nontoxic pharmaceutically acceptable carriers, adjuvants, and vehicles as desired. The term parenteral includes subcutaneous, intravenous, intramuscular, intrathecal, intrasternal injection, or infusion techniques.

The daily dose of the 1,2-dihydropyridine compounds of the invention (3-(2-cyanophenyl)-5-(2-pyridyl)-1-phenyl-1,2-dihydropyridin-2-one) is usually about 30 µg to 10 grams, preferably, 100 µg to 5 grams or, more preferably, 100 µg to 100 mg in the case of oral administration. For administration by injection, the daily dose is usually about 30 µg to 1 gram, preferably 100 µg to 500 mg or, more preferably, 100 µg to 30 mg. The compound is administered once daily or in several portions a day. When used in the context of a dosage amount, the numerical weight refers to the weight of the 1,2-dihydropyridine compound of the invention, exclusive of any salt, counterion, hydrate, and the like. Therefore to obtain the equivalent of 500 milligrams of 3-(2-cyanophenyl)-5-(2-pyridyl)-1-phenyl-1,2-dihydropyridin-2-one, it would be necessary to use more than 500 milligrams of a pharmaceutically acceptable salt and/or hydrate of the compound, due to the additional weight of the pharmaceutically acceptable salt and/or hydrate.

The daily dose of donepezil is usually about 0.1 milligram to 100 milligrams, preferably 1 milligram to 50 milligrams, more preferably 5 milligrams to 25 milligrams. In other embodiment, the daily dose is from 10 milligrams to 20 milligrams; or from 5 milligrams to 10 milligrams. The compounds are administered once daily or in several portions a day. When used in the context of a dosage amount, the numerical weight refers to the weight of the donepezil, exclusive of any salt, counterion, and the like. Therefore to obtain the equivalent of 10 milligrams of donepezil, it would be necessary to use more than 10 milligrams of donepezil hydrochloride, due to the additional weight of the hydrochloride.

In one embodiment, the mode of administration is by injection, such as subcutaneous injection, intramuscular injection, intravenous injection, or intra-arterial injection. Injectable preparations, for example, sterile injectable aqueous or oleaginous suspensions can be formulated according to the art using suitable dispersing or wetting agents, suspending agents (e.g., methylcellulose, Polysorbate 80, hydroxymethyl-cellulose, acacia, powdered tragacanth, sodium carboxymethylcellulose, polyoxyethylene sorbitan monolaurate and the like), pH modifiers, buffers, solubilizing agents (e.g., polyoxyethylene hydrogenated castor oil, Polysorbate 80, nicotinamide, polyoxyethylene sorbitan monolaurate, Macrogol, an ethyl ester of castor oil fatty acid, and the like) and preservatives. The sterile injectable preparation can also be a sterile injectable solution or suspension in a nontoxic parenterally acceptable diluent or solvent, for example, as a solution in 1,3-butanediol. Among the acceptable vehicles and solvents that can be used are water, Ringer's solution, and isotonic sodium chloride solution. In addition, sterile, fixed oils are conventionally used as a solvent or suspending medium. For this purpose any bland fixed oil can be used including synthetic mono- or diglycerides, in addition, fatty acids, such as oleic acid, can be used in the preparation of injectables. The preparations can be lyophilized by methods known in the art.

Solid dosage forms for oral administration can include chewing gum, capsules, tablets, sublingual tablets, powders, granules, and gels. In such solid dosage forms, the active compound can be admixed with one or more inert diluents such as lactose or starch. As is normal practice, such dosage forms can also comprise other substances including lubricating agents such as magnesium stearate. In the case of capsules, tablets, and pills, the dosage forms can also comprise buffering agents. The tablets can be prepared with enteric or film coatings, preferably film coatings.

To make tablets, the compounds can be admixed with pharmaceutically acceptable carriers known in the art such as, for example, vehicles (e.g., lactose, white sugar, mannitol, glucose, starches, calcium carbonate, crystalline cellulose, silicic acid, and the like), binders (e.g., water, ethanol, myranol, glucose solution, starch solution, gelatin solution, polyvinylpyrrolidone, and the like), disintegrators (e.g., dry starch, sodium, alginate, sodium hydrogen carbonate, calcium carbonate, polyoxyethylene sorbitan fatty acid esters, sodium laurylsulfate, stearic monoglyceride, starches, lactose, and the like), absorption promoters (e.g., quaternary ammonium base, sodium laurylsulfate, and the like), wetting agents (e.g. glycerin, starches, and the like), lubricants (e.g., stearates, polyethylene glycol, and the like), and flavoring agents (e.g., sweeteners). The tablets can be in the form of a conventional tablet, a molded tablet, a wafer and the like.

Sublingual administration refers to the administration in the mouth (e.g., under the tongue, between the cheek and gum, between the tongue and roof of the mouth). The highly vascular mucosal lining in the mouth is a convenient location for the compounds to be administered into the body.

In other embodiments, the solid dosage form can be packaged as granules or a powder in a pharmaceutically acceptable carrier, where the granules or powder are removed from the packaging and sprinkled on food or mixed with a liquid, such as water or juice, or where the granules are inserted into capsules. In this embodiment, the compounds described herein can be mixed with flavoring or sweetening agents. The packaging material can be plastic, coated paper, or any material that prevents water or moisture from reaching the granules and/or powder.

Liquid dosage forms for oral administration can include pharmaceutically acceptable emulsions, solutions, sublingual solutions, suspensions, and syrups containing inert diluents commonly used in the art, such as water. Such compositions can also comprise adjuvants, such as wetting agents, emulsifying and suspending agents, and sweetening, flavoring, and perfuming agents. To make sublingual solutions, the compounds can be admixed with various carriers, excipients, pH adjusters, and the like (e.g., water, sugar, lactic acid, acetic acid, fructose, glucose, saccharin, polyethylene glycol, propylene glycol, alcohol, bentonite, tragacanth, gelatin, alginates, aspartame, sorbitol, methylparaben, propylparaben, sodium benzoate, artificial flavoring and coloring agents).

For administration by inhalation, the compounds can be delivered from an insufflator, a nebulizer or a pressured pack or other convenient mode of delivering an aerosol spray. Pressurized packs can include a suitable propellant. Alternatively, for administration by inhalation, the compounds can be administered in the form of a dry powder composition or in the form of a liquid spray.

Suppositories for rectal administration can be prepared by mixing the active compounds with suitable nonirritating excipients such as cocoa butter and polyethylene glycols that are solid at room temperature and liquid at body temperature. Alternatively, an enema can be prepared by for rectal administration of the compounds described herein.

For topical administration to the epidermis, the compounds can be formulated as ointments, creams or lotions, or as the active ingredient of a transdermal patch. The compounds can also be administered via iontophoresis or osmotic pump. Ointments, creams and lotions can be formulated with an aqueous or oily base with the addition of suitable thickening and/or gelling agents. Alternatively, ointments, creams and lotions can be formulated with an aqueous or oily base and can also contain one or more emulsifying agents, stabilizing agents, dispersing agents, suspending agents, thickening agents, and/or coloring agents. As creams or lotions, the compounds can be mixed to form a smooth, homogeneous cream or lotion with, for example, one or more of a preservative (e.g., benzyl alcohol 1% or 2% (wt/wt)), emulsifying wax, glycerin, isopropyl palmitate, lactic acid, purified water, sorbitol solution. Such topically administrable compositions can contain polyethylene glycol 400. To form ointments, the compounds can be mixed with one or more of a preservative (e.g., benzyl alcohol 2% (wt/wt)), petrolatum, emulsifying wax, and Tenox (II) (e.g., butylated hydroxyanisole, propyl gallate, citric acid, propylene glycol). Woven pads or rolls of bandaging material, e.g., gauze, can be impregnated with the transdermally administrable compositions for topical application.

The compounds can also be topically applied using a transdermal system, such as one of an acrylic-based polymer adhesive with a resinous crosslinking agent impregnated with the compounds described herein and laminated to an impermeable backing. For example, the compounds can be administered in the form of a transdermal patch, such as a sustained-release transdermal patch. Transdermal patches can include any conventional form such as, for example, an adhesive matrix, a polymeric matrix, a reservoir patch, a matrix- or monolithic-type laminated structure, and are generally comprised of one or more backing layers, adhesives, penetration enhancers, and/or rate-controlling membranes. Transdermal patches generally have a release liner which is removed to expose the adhesive/active ingredient(s) prior to application. Transdermal patches are described in, for example, U.S. Patent Nos. 5,262,165, 5,948,433, 6,010,715 and 6,071,531, the disclosures of which are incorporated by reference herein in their entirety.

The invention provides for the compounds to be administered nasally to a patient to treat the diseases and disorders described herein. "Administered nasally" or "nasal administration" is intended to mean that at least one compound is combined with a suitable delivery system for absorption across the nasal mucosa of a patient. Generally, lower doses of the compound can be used for nasal administration when compared, for example, to the dose required for the oral administration.

The compounds can be administered, for example, as nasal sprays, nasal drops, nasal suspensions, nasal gels, nasal ointments, nasal creams or nasal powders. The compounds can also be administered using nasal tampons or nasal sponges. The compounds can be brought into a viscous basis via systems conventionally used, for example, natural gums, methylcellulose and derivatives, acrylic polymers (carbopol) and vinyl polymers (polyvinylpyrrolidone). In the compositions, many other excipients known in the art can be added such as water, preservatives, surfactants, solvents, adhesives, antioxidants, buffers, bio-adhesives, viscosity enhancing agents and agents to adjust the pH and the osmolarity.

The nasal delivery systems can take various forms including aqueous solutions, non-aqueous solutions and combinations thereof. Aqueous solutions include, for example, aqueous gels, aqueous suspensions, aqueous liposomal dispersions, aqueous emulsions, aqueous microemulsions and combinations thereof. Non-aqueous solutions include, for example, non-aqueous gels, non-aqueous suspensions, non-aqueous liposomal dispersions, non-aqueous emulsions, non-aqueous microemulsions and combinations thereof.

In other embodiments, the nasal delivery system can be a powder formulation. Powder formulations include, for example, powder mixtures, powder microspheres, coated powder microspheres, liposomal dispersions and combinations thereof. Preferably, the powder formulation is powder microspheres. The powder microspheres are preferably formed from various polysaccharides and celluloses selected from starch, methylcellulose, xanthan gum, carboxymethylcellulose, hydroxypropyl cellulose, carbomer, alginate polyvinyl alcohol, acacia, chitosans, and mixtures of two or more thereof.

In certain embodiments, the particle size of the droplets of the aqueous and/or non-aqueous solution or of the powders delivered to the nasal mucosa can be, for example, about 0.1 micron to about 100 microns; from about 1 micron to about 70 microns; from about 5 microns to about 50 microns; or from about 10 microns to about 20 microns. The particle sizes can be obtained using suitable containers or metering devices known in the art. Exemplary devices include mechanical pumps in which delivery is made by movement of a piston; compressed air mechanisms in which delivery is made by hand pumping air into the container, compressed gas (e.g., nitrogen) techniques in which delivery is made by the controlled release of a compressed gas in the sealed container; liquefied propellant techniques in which a low boiling liquid hydrocarbon (e.g., butane) is vaporized to exert a pressure and force the composition through the metered valve; and the like. Powders may be administered, for example, in such a manner that they are placed in a capsule that is then set in an inhalation or insufflation device. A needle is penetrated through the capsule to make pores at the top and the bottom of the capsule and air is sent to blow out the powder particles. Powder formulation can also be administered in a jet-spray of an inert gas or suspended in liquid organic fluids.

Also described herein is a nasally administrable pharmaceutical composition comprising at least one compound dispersed in a nasal delivery system that improves the solubility of the compound. The nasal delivery system that improves solubility can include one of the following or combinations thereof: (i) a glycol derivative (e.g., propylene glycol, polyethylene glycol, mixtures thereof); (ii) a sugar alcohol (e.g., mannitol, xylitol, mixtures thereof); (iii) glycerin; (iv) a glycol derivative (e.g., propylene glycol, polyethylene glycol or mixtures thereof) and glycerin; (v) ascorbic acid and water, (vi) sodium ascorbate and water; or (vii) sodium metabisulfite and water.

Also described herein is a nasally administrable pharmaceutical composition comprising at least one compound described herein and a nasal delivery system, where the nasal delivery system comprises at least one buffer to maintain the pH of the compound described herein, at least one pharmaceutically acceptable thickening agent and at least one humectant. The nasal delivery system can optionally further comprise surfactants, preservatives, antioxidants, bio-adhesives, pH adjusting agents, isotonicity agents, solubilizing agents, and/or other pharmaceutically acceptable excipients. The compound described herein can optionally be dispersed in a nasal delivery system that improves its solubility.

Also described herein is a nasally administrable pharmaceutical composition comprising at least one compound described herein and a nasal delivery system, where the nasal delivery system comprises at least one solubilizing agent, at least one pharmaceutically acceptable thickening agent and at least one humectant. The nasal delivery system can optionally further comprise buffers, pH adjusting agents, isotonicity agents, surfactants, preservatives, antioxidants, bio-adhesives, and/or other pharmaceutically acceptable excipients. The compound described herein can optionally be dispersed in a nasal delivery system that improves its solubility.

Also described herein is a nasally administrable pharmaceutical composition comprising at least one compound described herein and a nasal delivery system, where the nasal delivery system comprises at least one buffer to maintain the pH of the compound, at least one pharmaceutically acceptable thickening agent, at least one humectant, and at least one surfactant The nasal delivery system can optionally further comprise pH adjusting agents, isotonicity agents, solubilizing agents, preservatives, antioxidants, bio-adhesives, and/or other pharmaceutically acceptable excipients. The compound described herein can optionally be dispersed in a nasal delivery systems that improves its solubility.

Also described herein is a nasally administrable pharmaceutical composition comprising at least one compound described herein and a nasal delivery system, where the nasal delivery system comprises at least one pharmaceutically acceptable thickening agent, at least one humectant, at least one surfactant, and at least one solubilizing agent The nasal delivery system can optionally further comprise buffers, pH adjusting agents, isotonicity agents, preservatives, antioxidants, bio-adhesives, and/or other pharmaceutically acceptable excipients. The compound can optionally be dispersed in a nasal delivery system that improves its solubility.

Also described herein is a nasally administrable pharmaceutical composition comprising at least one compound described herein and a nasal delivery system, where the nasal delivery system comprises at least one buffer to maintain the pH of the compound, at least one pharmaceutically acceptable thickening agent, at least one humectant, at least one surfactant, and at least one solubilizing agent The nasal delivery system can optionally further comprise buffers, pH adjusting agents, isotonicity agents, preservatives, antioxidants, bio-adhesives, and/or other pharmaceutically acceptable excipients. The compound described herein can optionally be dispersed in a nasal delivery system that improves its solubility.

The buffer has a pH that is selected to optimize the absorption of the 1,2-dihydropyridine compound across the nasal mucosa. The particular pH of the buffer can vary depending upon the particular nasal delivery formulation as well as the specific compound selected. Buffers that are suitable for use in the invention include acetate (e.g., sodium acetate), citrate (e.g., sodium citrate dihydrate), phthalate, borate, prolamine, trolamine, carbonate, phosphate (e.g., monopotassium phosphate, disodium phosphate), and mixtures of two or more thereof.

The pH of the compositions should be maintained from about 3.0 to about 10.0. Compositions having a pH of less than about 3.0 or greater than about 10.0 can increase the risk of irritating the nasal mucosa of the patient Further, it is preferable that the pH of the compositions be maintained from about 3.0 to about 9.0. With respect to the non-aqueous nasal formulations, suitable forms of buffering agents can be selected such that when the formulation is delivered into the nasal cavity of a mammal, selected pH ranges are achieved therein upon contact with, e.g., a nasal mucosa.

The solubilizing agent for use in the compositions of the invention can be any known in the art, such as carboxylic acids and salts thereof. Exemplary carboxylic acid salts include acetate, gluconate, ascorbate, citrate, fumurate, lactate, tartrate, maleate, maleate, succinate, or mixtures of two or more thereof.

The viscosity of the compositions of the present invention can be maintained at a desired level using a pharmaceutically acceptable thickening agent For example, the viscosity may be at least 1000 cps; from about 1000 to about 10,000 cps; from about 2000 cps to about 6500 cps; or from about 2500 cps to about 5000 cps. Thickening agents that can be used in accordance with the present invention include, for example, methyl cellulose, xanthan gum, carboxymethyl cellulose, hydroxypropyl cellulose, carbomer, polyvinyl alcohol, alginates, acacia, chitosans, and mixtures of two or more thereof. The concentration of the thickening agent will depend upon the agent selected and the viscosity desired. Such agents can also be used in a powder formulation.

The nasally administrable compositions can also include a humectant to reduce or prevent drying of the mucus membrane and to prevent irritation thereof. Suitable humectant that can be used include, for example, sorbitol, mineral oil, vegetable oil and glycerol; soothing agents; membrane conditioners; sweeteners; and mixtures of two or more thereof. The concentration of the humectant will vary depending upon the agent selected. In one embodiment, the humectant can be present in the nasal delivery system in a concentration ranging from about 0.01 % to about 20% by weight of the compositions.

The nasal delivery system can further comprise surfactants which enhance the absorption of the compound. Suitable surfactants include non-ionic, anionic and cationic surfactants. Exemplary surfactants include oleic acid, polyoxyethylene derivatives of fatty acids, partial esters of sorbitol anhydride, such as for example, Tweens (e.g., Tween 80, Tween 40, Tween 20), Spans (e.g., Span 40, Span 80, Span 20), polyoxyl 40 stearate, polyoxy ethylene 50 stearate, fusieates, bile salts, octoxynol, and mixtures of two or more thereof. Exemplary anionic surfactants include salts of long chain hydrocarbons (e.g., C6-30 or C 10-20) having one or more of the following functional groups: carboxylates; sulfonates; and sulfates. Salts of long chain hydrocarbons having sulfate functional groups are preferred, such as sodium cetostearyl sulfate, sodium dodecyl sulfate and sodium tetradecyl sulfate. One particularly preferred anionic surfactant is sodium lauryl sulfate (i.e., sodium dodecyl sulfate). The surfactants can be present in an amount from about 0.001% to about 50% by weight, or from about 0.001% to about 20% by weight

The pharmaceutical compositions of the invention may further comprise an isotonicity agent, such as sodium chloride, dextrose, boric acid, sodium tartrate or other inorganic or organic solutes.

The nasal pharmaceutical compositions described herein can optionally be used in combination with a pH adjusting agent. Exemplary pH adjusting agents include sulfuric acid, sodium hydroxide, hydrochloric acid, and the like.

To extend shelf life, preservatives can be added to the nasally administrable compositions. Suitable preservatives that can be used include benzyl alcohol, parabens, thimerosal, chlorobutanol, benzalkonium chloride, or mixtures of two or more thereof. Preferably benzalkonium chloride is used. Typically, the preservative will be present in a concentration of up to about 2% by weight. The exact concentration of the preservative, however, will vary depending upon the intended use and can be easily ascertained by one skilled in the art.

Other ingredients which extend shelf life can be added such as for example, antioxidants. Some examples of antioxidants include sodium metabisulfite, potassium metabisulfite, ascorbyl palmitate and the like. Typically, the antioxidant will be present in the compositions in a concentration of from about 0.00 1 % up to about 5% by weight of the total composition.

The nasal delivery systems can be made following the processes described in, for example, U.S. Patent Nos. 6,451,848, 6,436,950, and 5,874,450, and WO 00/00199, the disclosures of which are incorporated by reference herein in their entirety.

### Examples

The following examples are for purposes of illustration only and are non intended to limit the spirit or scope of the claims.

### Example 1

The role of dopamine in cognition, emotion and motor performance has been studied. It was reported that there was a link between age related nigrostriatal dopaminergic decline and age-related decline of cognitive function. Erixon-Lindrotha et al, Psychiatry Research: Neuroimaging, 138:1-12 (2005). It was also reported that pharmacological manipulation of dopamine systems could modify cognitive performance in humans. Luciana et al, Cereb. Cortex, 8:218-226 (1998); Kimberg et al, Neuropsychologia, 41:1020-1027 (2003). Facilitation of dopaminergic activity could improve cognitive function in human.

Acetylcholinesterase inhibitors are often utilized for the treatment of dementia. It was reported that donepezil can increase the release of dopamine in the brain, and it was also reported that coadministration with the MAO-B inhibitor selegilline enhanced learning ability. Giacobini et all Neuropharmacology, 35:205-211 (1996); Takahata et al, Eur. J. Pharmacol., 518:140-144 (2005). It was reported that other drugs for Alzheimer's disease (e.g., memantine) have some effect on dopamine turnover and are effective in combination with donepezil. Bubser et al, Eur. J. Pharmacol., 229:75-82 (1992); Tariot et al, JAMA, 291:317-324 (2004). These results indicated that improvement of cognitive function by acetylcholinesterase inhibitors can be augmented by compounds having positive effects on dopaminergic activity.

C57BL/6 mice (SLC, Shizuoka, Japan) were used for the experiment. 3-(2-cyanophenyl)-5-(2-pyridyl)-1-phenyl-1,2-dihydropyridin-2-one and donepezil were suspended or dissolved in a 0.5% methyl cellulose solution. The two drugs were orally administered by gavage 1 hour before sampling. At the time the mice were sacrificed, both striata were rapidly dissected and were frozen by liquid nitrogen. The samples were subsequently sonicated in 0.5 ml of chilled 0.5 M perchloric acid containing 0.1 M EDTA, and centrifuged at 13000 rpm for 15 min at 4°C. Then monoamine levels were measured by HPLC with electrochemical detection. Supernatant aliquots of 20 µl were separated over an Eicompak SC5-ODS column (3 x 150 mm; Eicom, Japan) with mobile phase of 17% methanol, 83% 0.1M acetate/citrate buffer (pH 3.5) containing 190 mg/ml sodium 1-octanesulfonate and 50 mg/ml EDTA, and a flow rate of 0.65 ml/min. Peaks of dopamine, DOPAC, HVA, 5-HT, and 5- HIAA were quantified by area integration with PowerChrom (eDAQ, Australia), and concentrations were expressed as nanograms per milligram of striatal tissue.

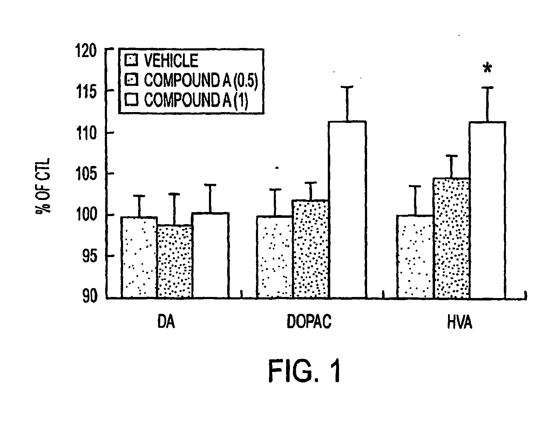

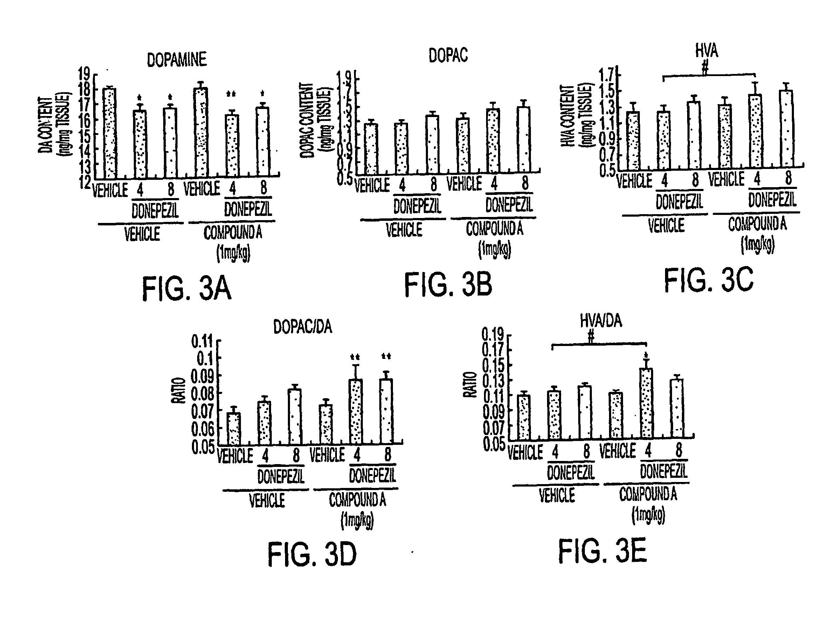

As shown in Figure 1, 3-(2-cyanophenyl)-5-(2-pyridyl)-1-phenyl-1,2-dihydropyridin-2-one (i.e., Compound A) elevated the content of HVA, which is a metabolite of dopamine in striatum at 1mg/kg.

### Example 2

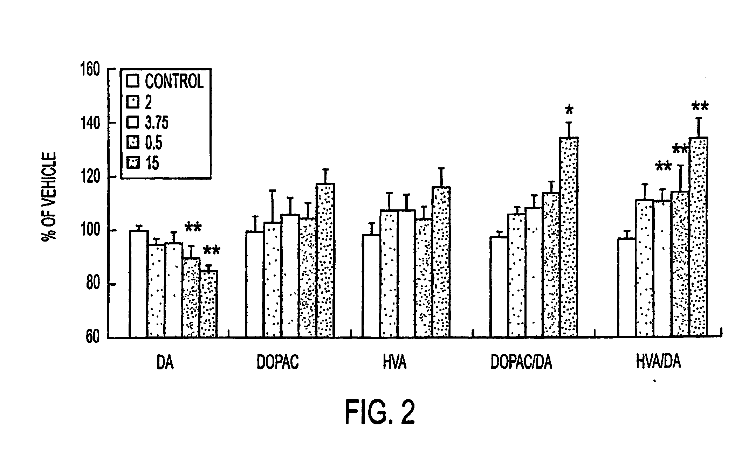

Donepezil reduced the content of dopamine significantly but elevation of dopamine metabolite did not reach significance. Figure 2 shows that DPAC/DA and HVA/DA, which also indicate increased dopamine turnover, increased significantly. Behavioral symptoms of donepezil were observed in the experiment. Donepezil treated mice showed peripheral side effect at 7.5 mg/kg and higher. These results indicate that both 3-(2-cyanophenyl)-5-(2-pyridyl)-1-phenyl-1,2-dihydropyridin-2-one monotherapy and donepezil monotherapy modulated dopamine turnover but neither reached significance, suggesting a modest effect on dopamine turnover. Figure 3, however, shows that the combination of 3-(2-cyanophenyl)-5-(2-pyridyl)-1-phenyl-1,2-dihydropyridin-2-one and donepezil unexpectedly facilitated superior dopamine turnover in mice compared to monotherapy with the individual compounds. This experiment demonstrates that the combination therapy of 3-(2-cyanophenyl)-5-(2-pyridyl)-1-phenyl-1,2-dihydropyridin-2-one and donepezil (when compared to monotherapy with either 3-(2-cyanophenyl)-5-(2-pyridyl)-1-phenyl-1,2-dihydropyridin-2-one or donepezil) can be used to treat cognitive dysfunctions, dementia, and neurodegernative diseases, such as Alzheimer's disease.

### Example 3

Example 5 shows the neuroprotective effects of 3-(2-cyanophenyl)-5-(2-pyridyl)-1-phenyl-1,2-dihydropyridin-2-one and donepezil. A combination of 3-(2-cyanophenyl)-5-(2-pyridyl)-1-phenyl-1,2-dihydropyndin-2-one and donepezil show clear neuroprotective effects against excitotoxicity; however, the profile of both drugs is different. Therefore, complementary approaches to maximize the neuroprotective effects by the combination of drugs may be required to halt progression of neurodegenerative diseases including Alzheimer's disease, Parkinson's disease, Huntington's disease, amyotrophic lateral sclerosis and the like.

It has been described that neurodegenerative diseases exhibit excitotoxicity in the process of neurodegeneration. Rego et al, Neurochemical Research, 28:1563-1574 (2003). 3-(2-cyanophenyl)-5-(2-pyridyl)-1-phenyl-1,2-dihydropyridin-2-one is AMPA antagonist. AMPA antagonists have an effect on excitotoxic conditions induced by kainate and metabolic stress. Ohno et al, JPET, 306:66-72 (2003); Matsuoka et al, Neuroreport, 6:2205-2208 (1995). On the other hand, it is known that AMPA antagonists show no and limited protective effects against NMDA-induced toxicity. Kristensen, Brain Res., 917:21-44 (2001).

Donepezil, commercially available as ARICEPT® (Eisai, Inc., Teaneck, NJ) is prescribed for Alzheimer's disease. Donepezil improves cognition in patients with dementia, which is caused by acetylcholinesterase inhibition. Donepezil has exhibits neuroprotective effects against excitotoxicity induced by NMDA, but not by kainate. Akasofu et al, Eur. J. Pharmacol., 530:215-222 (2006). The neuroprotective effects of donepezil are supported by clinical findings in brain atrophy of patients with Alzheimer's disease. Hashimoto et al, Am J. Psychiatry,162:676-682 (2005). Donepezil could be a disease modifying drug for neurodegenerative diseases; however, the neuroprotective effects of donepezil are insufficient to completely halt the progression of Alzheimer's disease.

Combination therapy is sometimes beneficial but sometimes harmful. Therefore it is very important to confirm the safety and efficacy of the combination. The combination therapy of the two drugs (3-(2-cyanophenyl)-5-(2-pyridyl)-1-phenyl-1,2-dihydropyridin-2-one and donepezil) in cultured neurons enhanced the beneficial effects against NMDA- and kainate-induced neurodegeneration. Neurodegeneration by the drug combination was not observed. Therefore, the combination of drugs can be a favorable option for the treatment of neurodegeneration, which leads to diseases like Alzheimer's disease, Parkinson's disease, Huntington's disease, amyotrophic lateral sclerosis, and the like.

Cortical cell cultures were prepared from fetal rats of the Wistar strain (gestational age of 17-19 days, Charles River Japan, Kanagawa, Japan). The cortex was dissected and kept in ice-cold Neurobasal medium* (( Neurobasal medium (Gibco BRL) containing 2% B27 supplement ((Gibco BRL), 0.5mM L-glutamine(Gibco BRL), 2% Antibiotic-Antimycotic liquid(Gibco BRL), 25µM 2-mercaptoethanol)) and then incubated at 37°C for 30 min in Ca²⁺/Mg²⁺-free HBSS containing 0.25% trypsin (Gibco BRL) and 0.2 mg/ml deoxyribonuclease (DNase) (Sigma, St. Louis, MO, USA). The cortical tissues were dissociated to single cells by gentle trituration. The cell suspension was mixed with Neurobasal medium with 10% heat-inactivated fetal calf serum (Gibco BRL). The cell suspension was centrifuged at 3 10 x g for 3 min and the resulting pellets were resuspended in the medium described above.

Cells were plated at an initial cell density of 2.0 x 10⁵ cells/cm² in poly-L-lysine-coated well plates. The cells were cultured in a CO₂ incubator 5% (v/v), at 37°C in Neurobasal medium containing 2% B27 supplement for 7 days.

The cultures were exposed to 200 µM NMDA (Sigma) or 200 µM kainite in neurobasal medium containing 2% B27 supplement(-AO) for 24 h at 37°C. The cells were treated with donepezil and 3-(2-cyanophenyl)-5-(2-pyndyl)-1-phenyl-1,2-dihydropyridin-2-one for 24 hours before and during the exposure to these agonists.

LDH efflux into the culture medium was measured after NMDA or kainate insult. Remaining LDH in the cells was measured after lysing the cells with 0.1 M phosphate buffer (0.1 M Na₂HPO₄ and 0.1 M KH₂PO₄: pH 7.4) containing 0.5% Triton X-100. LDH release as an indicator of neuronal injury was presented as the percentage leakage of LDH into the culture medium, with respect to the total LDH activity per culture (LDH efflux/total LDH measured). The decrease of NADH was spectrophotometrically monitored at 340 nm as a measure of LDH activity.

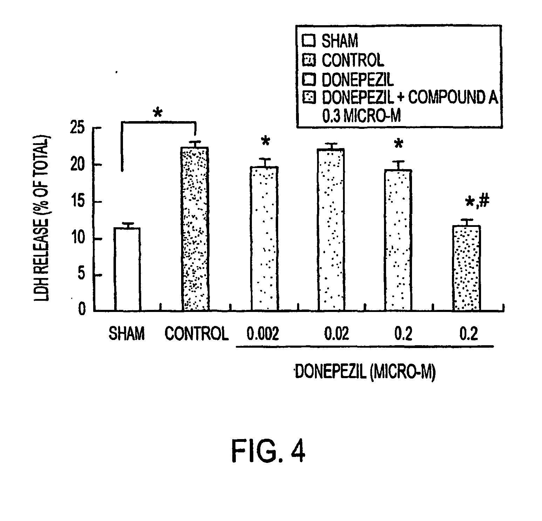

In kainate-induced neuronal toxicity, donepezil showed modest effect on neuronal survival. Figure 4 shows that the co-administration of donepezil and 3-(2-cyanophenyl)-5-(2-pyridyl)-1-phenyl-1,2-dihydropyridin-2-one significantly and unexpectedly enhanced neuronal survival compared to donepezil alone.

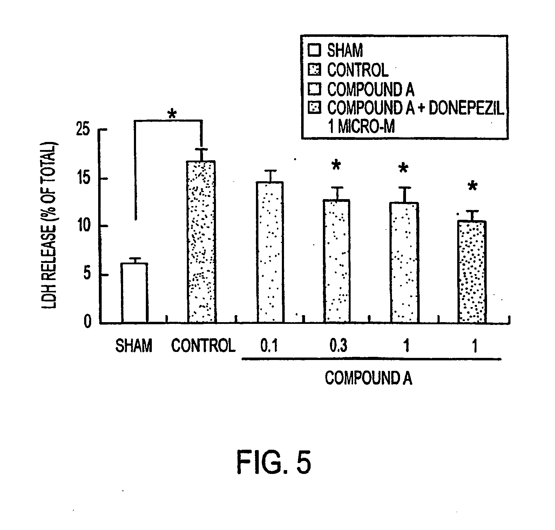

In the NMDA experiment, 3-(2-cyanophenyl)-5-(2-pyridyl)-1-phenyl-1,2-dihydropyridin-2-one showed effects and reached a plateau from 0.3 mM. As shown in Figure 5, the co-administration of 3-(2-cyanophenyl)-5-(2-pyridyl)-1-phenyl-1,2-dihydropyridin-2-one and donepezil unexpectedly and significantly enhanced neuronal survival.

### Example 4

Organotypic hippocampal slice cultures were prepared using the basic method of Pringle et al, Brain Research, 755:36-46 (1997) modified as follows. Wistar rat pups (8-11 days old) were decapitated and the hippocampus rapidly dissected into ice-cold Gey's balanced salt solution supplemented with 4.5 mg/ml glucose. Transverse sections (400 µm) were cut on a McIlwain tissue chopper and placed back into ice-cold Gey's balanced salt solution. Slices were separated and plated onto Millicell CM culture inserts (4 per well) and maintained at 37°C/5% CO₂ for 14 days. Maintenance medium consisted of 25% heat-inactivated bone serum, 25% Hank's balanced salt solution (HBSS) and 50% minimum essential medium with added Earle's salts (MEM) supplemented with 1 mM glutamine and 4.5 mg/ml glucose. Medium was changed every 3-4 days.

Cultures were transferred to serum free medium (SFM - 75% MEM, 25% HBSS) supplemented with 1mM glutamine and 4.5mg/ml glucose) containing 5 µg/ml of the fluorescent exclusion dye propidium iodide (PI), and allowed to equilibrate for 60 minutes prior to imaging. PI fluorescence was detected using a Leica DMIL inverted microscope fitted with a rhodamine filter set. Any cultures in which PI fluorescence was detected at this stage were excluded from further study. 3-(2-cyanophenyl)-5-(2-pyridyl)-1-phenyl-1,2-dihydropyridin-2-one and/or donepezil HCl was added to the SFM and cultures returned to the incubator for 24 hours. After this time, cultures were exposed to a neurotoxic insult (see below for protocols) in the presence of PI and 3-(2-cyanophenyl)-5-(2-pyridyl)-1-phenyl-1,2-dihydropyridin-2-one and/or donepezil HCl before being returned to SFM containing 3-(2-cyanophenyl)-5-(2-pyridyl)-1-phenyl-1,2-dihydropyridin-2-one and/or donepezil HCl. Neuronal damage was assessed by PI fluorescence imaging 24 hours after the initiation of the neurotoxic insult. Neurotoxicity protocols are outlined below.
(1) AMPA: Cultures were transferred to fresh SFM (+PI) containing 30 J.1M S-AMPA for 3 hours under normal culture conditions. At the end of this period, cultures were returned to fresh SFM for 21 hours prior to determination of neuronal death by PI imaging. GYKI52466 (30 µM) was used as a positive, neuroprotective control.
(2) Oxygen-Glucose Deprivation (OGD): Experimental ischaemia was performed by exposing cultures to oxygen-glucose deprivation (OGD) as described previously. Pringle et al, Brain Research, 755:36-46 (1997); Pringle et al, Stroke, 27:2124-2130 (1996); Morrison et al, Br. J. Pharmacol., 137:1255-1268 (2002). OGD was induced by transferring cultures to glucose-free SFM (+PI) which had been saturated with 95%N₂/5%CO₂. Culture plates (without lids) were then sealed into an airtight chamber in which the atmosphere was saturated with 95%N₂/5%CO₂ by continuously blowing through gas at 10 L/min for ten minutes before being sealed and placed in the incubator for 50 mins (total time of OGD was therefore 60 mins). At the end of the period of OGD cultures were returned to normoxic SFM containing PI and placed back in the incubator for 23 hours.
(3) Hypoxia: Hypoxia was performed by exposing cultures to oxygen-deprivation as described previously. Pringle et al, Brain Research, 755:36-46 (1997); Pringle et al, Stroke, 27:2124-2130 (1996). Hypoxia was induced by transferring cultures to SFM (+PI) which had been saturated with 95%N₂/5%CO₂. Culture plates (without lids) were then sealed into an airtight chamber in which the atmosphere was saturated with 95%N₂/5%CO₂ by continuously blowing through gas at 10 L/min for ten minutes before being sealed and placed in the incubator for 170 mins (total time of hypoxia was therefore 180 mins). At the end of the period of hypoxia, cultures were returned to normoxic SFM (+PI) and placed back in the incubator for 21 hours. The freeradical scavenger MnTBAP (100 µM) was used as a positive neuroprotective control.
(4) NMDA: Cultures were transferred to fresh SFM (+PI) containing 10 µM NMDA for 3 hours under normal culture conditions. At the end of this period, cultures were returned to fresh SFM for 21 hours prior to determination of neuronal death by PI imaging. MK801 (10 µM) was used as a positive, neuroprotective control.
(5) Free radical-mediated toxicity (Duroquinone): Duroquinone produces superoxide-mediated neurotoxicity producing a selective neuronal lesion in the CA1 region. Wilde et al, J. Neurochem., 69(2):883-886 (1997). Cultures were transferred to fresh SFM (+PI) containing 100 µM duroquinone for 3 hours under normal culture conditions. At the end of this period, cultures were returned to fresh SFM for 21 hours prior to determination of neuronal death by PI imaging. MnTBAP (100 µM) was used as a positive neuroprotective control.

Neuronal damage was assessed using ImageJ software running on a PC. Images were captured using a monochrome CCD camera and saved for otlline analysis. Light transmission images were captured prior to the addition of drugs, and PI fluorescence images recorded at the end of the 24-hour recovery period. The area of the CA1 region was determined from the transmission image. The area of PI fluorescence in the CA1 was measured using the threshold function on ImageJ, and neuronal damage expressed as the percentage of the CA1 in which PI fluorescence was detected above background. Morrison et al, Br. J. Pharmacol., 137:1255-1268 (2002).

MnTBAP and MK-801 were dissolved in distilled water to 10 mM. NMDA was dissolved in distilled water to 100 mM, and AMPA was dissolved in distilled water to 3 mM. GYKI52466 was dissolved in DMSO to 3 mM. Stock solutions (1 mM) of donepezil were prepared fresh each day in distilled water, and then serially diluted into SFM. Stock solutions of 3-(2-cyanophenyl)-5-(2-pyridyl)-1-phenyl-1,2-dihydropyridin-2-one (1 mM) were prepared fresh each day in DMSO and further diluted into SFM.

Materials were obtained from the following suppliers: tissue culture plates - Nunc (Fisher Scientific, Loughborough, UK); all other tissue culture plastics were purchased from - Bibby Sterilin (Stone, UK); culture media - Invitrogen (Paisley, UK); PI - Molecular Probes (Leiden, Holland); S-AMPA, GYKI52466, NMDA, MK801 and MnTBAP -Tocris (Bristol, UK); 3-(2-cyanophenyl)-5-(2-pyridyl)-1-phenyl-1,2-dihydropyridin-2-one, donepezil hydrochloride (Eisai Co. Ltd).

Data analysis was performed using GraphPad Prism. Data were initially assessed for normality after which statistical significance was determined using a one way analysis of variance followed by a Dunnett's post-hoc test. All data is expressed as mean ± s.e.m. Raw data and analysis of individual datesets are not provided herewith.

The EC50 for 3-(2-cyanophenyl)-5-(2-pyridyl)-1-phenyl-1,2-dihydropyridin-2-one in AMPA toxicity was determined. No PI fluorescence was observed in slice cultures maintained in SFM for up to 49 hours (sham controls). In contrast, significant neurodegeneation was observed in the CA1 region of cultures exposed to 30 µM AMPA for 3 hours (65.1 ± 1.7% damage in CA1, n=104, p<0.001 vs sham controls). AMPA-mediated neurodegeneration was significantly attenuated by the non-competitive AMPA receptor antagonist GYKI52466, which reduced PI fluorescence to 14.3 ± 2.3% damage (n=60, p<0.001 vs AMPA). AMPA-induced neurodegeneration was reduced in a concentration-dependent manna by 3-(2-cyanophenyl)-5-(2-pyridyl)-1-phenyl-1,2-dihydropyridin-2-one. At 0.1 µM 3-(2-cyanophenyl)-5-(2-pyridyl)-1-phenyl-1,2-dihydropyridin-2-one, no neuroprotective efficacy was observed (63.9 ± 2.6% damage, n=36, p=NS vs AMPA). When the concentration of 3-(2-cyanophenyl)-5-(2-pyridyl)-1-phenyl-1,2-dihydropyridin-2-one was increased to 0.3 µM, significant neuroprotection was observed, with damage reduced to 50.4 ± 2.5% (n=60, p<0.01 vs AMPA). At the highest concentration of 3-(2-cyanophenyl)-5-(2-pyridyl)-1-phenyl-1,2-dihydropyridin-2-one (3 µM), neuroprotection was almost complete (0.1 ± 0.1% damage, n=36, p<0.001 vs AMPA). Based on this series of experiments, the EC50 for 3-(2-cyanophenyl)-5-(2-pyridyl)-1-phenyl-1,2-dihydropyridin-2-one in this model of AMPA toxicity was determined to be 0.5 µM.

The effect of donepezil on AMPA-mediated toxicity was determined. Initially, a series of cultures (24 per group) was exposed to either SFM or donepezil (0.1 to 10 µM) for 48 hours (solutions were changed for ones containing flesh donepezil after 24 hours) to assess potential toxicity of the compound. No PI fluorescence was observed in any culture in this experiment indicating that the compound was nontoxic in this paradigm (data not shown).

A further set of preliminary experiments was performed in order to determine the effect of increasing the pre-incubation time (4 to 48 hours) of donepezil prior to exposure of cultures to AMPA. As previously, no PI fluorescence was observed in cultures maintained in SFM for the duration of the experiment, but significant toxicity was observed in CA1 following exposure to AMPA (46.5 ± 5.0% damage, n=24). This was significantly attenuated by 30 µM GYK152466 (21.7 ± 2.7% damage, n=24, p<0.001 vs AMPA). No effect of donepezil was observed in the longer pre-incubation times. PI 55.51 ± 2.6% of CA1 (24 hours pre-incubation, n=24, p=NS), respectively. However, when the pre-incubation time was decreased to 4 hours, a small but significant increase in AMPA-mediated toxicity was observed, with PI fluorescence being observed in 57.4 ± 2.3% of CA1 (n=24, p<0.05 vs AMPA).

No biologically significant effect was observed by altering the pre-incubation period; therefore, a standard pre-incubation period of 24 hours was chosen for the remainder of the experiments based on published evidence. Akasofu et al, Eur. J. Pharmacol., 472(1-2):57-63 (2003); Takada et al, Pharmacol. Exp. Ther., 306(2):772-777 (2003).

In a further series of experiments, exposure of cultures to 30 µM AMPA resulted in PI fluorescence occurring in 62.4 ± 2.4% of the CA1 region (n=72). This was significantly attenuated by 0.5 µM 3-(2-cyanophenyl)-5-(2-pyridyl)-1-phenyl-1,2-dihydropyridin-2-one, reducing the area of PI fluorescence to 33.6 ± 4.0% of CA1 (n=24, p<0.001 vs AMPA). No neuroprotective effect of donepezil was observed at any of the concentrations tested (0.1 to 10 µM). As observed previously (Figures 8 and 9), we observed a small, but non-significant increase in AMPA-mediated toxicity in the presence of all three concentrations of donepezil (0.1 µM: 69.5 ± 2.4% of CA1; 1 µM: 70.7 ± 2.4% of CA1; 10 µM: 70.9% ± 2.8% of CA1; all groups n=36, p=NS vs AMPA). Although 0.5 µM 3-(2-cyanophenyl)-5-(2-pyridyl)-1-phenyl-1,2-dihydropyridin-2-one was significantly neuroprotective when present alone, the efficacy of 3-(2-cyanophenyl)-5-(2-pyridyl)-1-phenyl-1,2-dihydropyridin-2-one was lost when the compound was co-incubated with donepezil. This loss of efficacy was statistically significant at all three concentrations of donepezil (0.1 µM donepezil + -(2-cyanophenyl)-5-(2-pyridyl)-1-phenyl-1,2-dihydropyridin-2-one: 70.2 ± 1.6% of CA1; 1 µM: 62.6 ± 3.6% of CA1; 10 µM: 68.7% ± 2.4% of CA1; all groups n=36, p<0.001 vs AMPA).

Experimental ischaemia was induced by exposing cultures to 60 minutes oxygen-glucose deprivation (OGD). An initial set of experiments was conducted to determine the neuroprotective efficacy of 3-(2-cyanophenyl)-5-(2-pyridyl)-1-phenyl-1,2-dihydropyridin-2-one against OGD-induced neurotoxicity. Exposure of cultures to 60 minutes OGD produced extensive PI fluorescence in the CA1 region (50.6 ± 5.2% damage, n=24) and this was prevented by the fee-radical scavenger MnTBAP (100 µM) (0.1 ± 0.0% damage, n=24, p<0.001 vs OGD). No significant neuroprotection was observed in cultures treated with the non-competitive AMPA receptor antagonist GYKI52466 (30 µM) (36.9 ± 3.6%, n=24, p=NS). In order to assess the EC50 for 3-(2-cyanophenyl)-5-(2-pyridyl)-1-phenyl-1,2-dihydropyridin-2-one, a six-point concentration-response curve was generated. Surprisingly, in cultures treated with the lowest concentration of 3-(2-cyanophenyl)-5-(2-pyndyl)-1-phenyl-1,2-dihydropyridin-2-one (0.1 µM), a significant increase in PI fluorescence was observed (64.4 ± 3.6%, n=24, p<0.05 vs OGD). There was no significant effect of 3-(2-cyanophenyl)-5-(2-pyridyl)-1-phenyl-1,2-dihydropyridin-2-one at any other concentration assessed (up to 3 µM).

As a separate EC50 could not be established in this model, further experiments were performed using 0.5 µM 3-(2-cyanophenyl)-5-(2-pyridyl)-1-phenyl-1,2-dihydropyridin-2-one, the EC50 for blockade of AMPA toxicity.

In a second series of experiments, the effects of donepezil were investigated on OGDinduced neurotoxicity, and how this was affected by 3-(2-cyanophenyl)-5-(2-pyridyl)-1-phenyl-1,2-dihydropyridin-2-one. In cultures exposed to OGD alone, PI fluorescence occurred in 44.8 ± 3.3% of the CA1 region (n=48). As previously, this was fully attenuated by 100 µM MnTBAP (0.6 ± 0.2% damage, n=48, p<0.001 vs OGD), but not by 0.5 µM 3-(2-cyanophenyl)-5-(2-pyridyl)-1-phenyl-1,2-dihydropyridin-2-one (55.3 ± 3.1% damage, n=32, p=NS). Pretreatment with donepezil (0.1 to 10 µM) had no effect on neuronal damage (0.1 µM: 49.4 ± 2.7% damage; 1 µM: 54.1 ± 4.7% damage; 10 µM: 45.3 ± 3.3%; all n=48, p=NS vs OGD). In cultures exposed to OGD following co-application of 1 µM donepezil and 0.5 µM 3-(2-cyanophenyl)-5-(2-pyridyl)-1-phenyl-1,2-dihydropyridin-2-one there was a small, but significant increase in PI fluorescence (64.3 ± 4.2% damage, n=48, p<0.05 vs OGD). No effect was observed at higher (10 µM) or lower (0.1 µM) concentrations of donepezil, suggesting that this effect may not be biologically relevant.

Duroquinone-induced injury was determined. Free radical-induced injury was produced by exposure of cultures to 100 µM duroquinone for 3 hours followed by a 21 hour recovery period. Cultures exposed to 100 µM duroquinone for 3 hours developed neurodegeneration in the CA1 region, resulting in extensive PI fluorescence (51.0 ± 3.4% damage, n-72). As expected, duroquinone-induced injury was fully abolished by the free radical scavenger MnTBAP (100 µM) (0.1 ± 0.0% damage, n=24, p<0.001 vs duroquinone). 3-(2-cyanophenyl)-5-(2-pyridyl)-1-phenyl-1,2-dihydropyridin-2-one (0.5 µM) was not neuroprotective in this model (69.1 ± 4.0% damage, n=24, p=NS). Donepezil did not reduce duroquinone-induced neuronal damage (0.1 µM: 56.4 ± 4.4% damage; 1 µM: 53.2 ± 4.9% damage; 10 µM: 56.6 ± 4.4% damage, all n=36, p=NS). Similarly, co-incubation with 3-(2-cyanophenyl)-5-(2-pyridyl)-1-phenyl-1,2-dihydropyridin-2-one and donepezil did not affect neuronal damage.

NMDA-induced neurotoxicity was investigated. Exposure of cultures to 10 µM NMDA for 3 hours produced neurodegeneration in the CA1 region as determined by a significant increase in PI fluorescence (35.1 ± 2.2% damage, n=72), and this was fully attenuated by the non-competitive NMDA receptor antagonist MK-801 (10 µM) (0.0 ± 0.0% damage, n=36, p<0.001 vs NMDA). In contrast, NMDA toxicity was unaffected by 3-(2-cyanophenyl)-5-(2-pyridyl)-1-phenyl-1,2-dihydropyridin-2-one (0.5 µM) (29.1 ± 2.9% damage, n=36, p=NS). Donepezil was significantly neuroprotective at all three concentrations tested in a concentration-dependent manner (0.1 µM: 23.0 ± 2.4% damage, p<0.01 vs NMDA; 1 µM: 25.2 ± 2.3% damage, p<0.05 vs NMDA; 10 µM: 14.5 ± 2.2% damage p<0.001 vs NMDA; all groups n=36). The neuroprotective efficacy of low concentrations of donepezil was attenuated by inclusion of 0.5 µM 3-(2-cyanophenyl)-5-(2-pyridyl)-1-phenyl-1,2-dihydropyridin-2-one (0.1 µM + 3-(2-cyanophenyl)-5-(2-pyridyl)-1-phenyl-1,2-dihydropyridin-2-one: 27.9 ± 2.6% damage; 1 µM + 3-(2-cyanophenyl)-5-(2-pyridyl)-1-phenyl-1,2-dihydropyridin-2-one: 29.5 ± 3.0% damage; both groups n=36). 10 µM donepezil remained neuroprotective in the presence of 3-(2-cyanophenyl)-5-(2-pyridyl)-1-phenyl-1,2-dihydropyridin-2-one (24.9 ± 2.4% damage, n=36, p<0.05 vs NMDA).

Hypoxia-induced neurodegeneration was examined. Hypoxia-mediated neuronal damage was produced by exposing cultures to oxygen deprivation for a period of 3 hours followed by a 21 hour recovery period. 3 hours hypoxia resulted in a lesion within CA1, as defined by PI fluorescence (45.1 ± 3.5% damage, n=96). This was prevented by the free-radical scavenger MnTBAP (100 µM) (0.2 ± 0.1% damage, n-48, p<0.001 vs hypoxia). Donepezil (0.1 to 10 µM) was highly neuroprotective in this model producing a significant reduction in neuronal damage at the 0.1 µM (31.7 ± 4.6% damage, n=48, p<0.05 vs hypoxia). Neuroprotective efficacy was increased when the concentration of donepezil was increased to 1 µM (14.9 ± 3.2% damage, n=48, p<0.00 1 vs hypoxia). No further increase in efficacy was observed when the concentration of donepezil was increased to 10 µM (12.9 ± 2.9% damage, n=48, p<0.001 vs hypoxia). A concentration-dependent neuroprotective effect of 3-(2-cyanophenyl)-5-(2-pyridyl)-1-phenyl-1,2-dihydropyridin-2-one was also observed, with significant efficacy being observed at 0.1 µM (24.8 ± 6.3% damage, n=24, p<0.001 vs hypoxia). At the highest concentration tested (5 µM 3-(2-cyanophenyl)-5-(2-pyridyl)-1-phenyl-1,2-dihydropyridin-2-one), the compound was almost as effective as MmTBAP, reducing damage to 3.9 ± 1.8% (n=24, p<0.001 vs hypoxia).

A second set of experiments was performed to investigate whether the neuroprotective effects of donepezil and 3-(2-cyanophenyl)-5-(2-pyridyl)-1-phenyl-1,2-dihydropyridin-2-one were synergistic or additive. In order to test the hypothesis, the lowest effective concentrations (i.e., 0.1 µM) of each of the two compounds was used). Hypoxia alone resulted in 36.4 ± 3.9% damage (n=48), and this was prevented by MnTBAP (100 µM) (0.0 ± 0.0%, n=24, p<0.001 vs hypoxia). Neither donepezil (0.1 µM: 30.4 ± 3.9% damage, n=48) nor 3-(2-cyanophenyl)-5-(2-pyridyl)-1-phenyl-1,2-dihydropyridin-2-one (0.1 µM: 36.7 ± 4.2% damage, n=48, p=NS) were neuroprotective in this series of experiments. Similarly, no reduction in PI fluorescence was observed in culture incubated with both compounds together (33.8 ± 4.1% damage, n=48, p=NS).

It is likely that 0.1 µM lies very close to the threshold concentration for neuroprotective efficacy for donepezil and 3-(2-cyanophenyl)-5-(2-pyridyl)-1-phenyl-1,2-dihydropyridin-2-one. The experiment using 0.5 µM donepezil and 0.5 µM 3-(2-cyanophenyl)-5-(2-pyridyl)-1-phenyl-1,2-dihydropyridin-2-one was repeated. In this experiment, exposure of cultures to hypoxia resulted in 36.9 ± 4.4% damage (n=48) and this was blocked by 100 µM MnTBAP (0.8 ± 0.4% damage, n=24, p<0.001 vs hypoxia). Donepezil (0.5 µM) produced a significant decrease in neuronal damage (25.3 ± 3.6% damage, n=48, p<0.05 vs hypoxia), an effect mirrored by 3-(2-cyanophenyl)-5-(2-pyridyl)-1-phenyl-1,2-dihydropyridin-2-one (0.5 µM) (27.1 ± 3.5% damage, n=48, p<0.05 vs hypoxia). A similar level of neuroprotection was observed when donepezil and 3-(2-cyanophenyl)-5-(2-pyridyl)-1-phenyl-1,2-dihydropyridin-2-one were used in combination (24.7 ± 3.5% damage, n=48, p<0.05 vs hypoxia).

Each of the patents, patent applications, and publications cited herein are incorporated by reference herein in their entirety.

It will be apparent to one skilled in the art that various modifications can be made to the invention without departing from the spirit or scope of the appended claims.

## Claims

1. A compound for use in combination with donepezil or a pharmaceutically acceptable salt thereof in the treatment of (i) Alzheimer's disease in a patient or (ii) dementia or one or more cognitive impairments in a patient; wherein the compound is 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof.

2. A compound for use in combination with 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof in the treatment of (i) Alzheimer's disease in a patient or (ii) dementia or one or more cognitive impairments in a patient; wherein the compound is donepezil or a pharmaceutically acceptable salt thereof.

3. The compound of claim 1 or 2, wherein donepezil or the pharmaceutically acceptable salt thereof, and 3-(2-cyanophenyl)-5-(2-pyridyl)-1-phenyl-1,2-dihydropyridin-2-one, the pharmaceutically acceptable salt thereof, the hydrate thereof or the hydrate of a pharmaceutically acceptable salt thereof are (a) administered separately to the patient or (b) administered to the patient in the form of a pharmaceutical composition.

4. The compound of any one of the preceding claims wherein the dementia is caused by Alzheimer's disease, Parkinson's disease, or Huntington's disease.

5. Use of 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof in the manufacture of a medicament for use in combination with donepezil or a pharmaceutically acceptable salt thereof in the treatment of (i) Alzheimer's disease in a patient or (ii) dementia or one or more cognitive impairments in a patient.

6. Use of donepezil or a pharmaceutically acceptable salt thereof in the manufacture of a medicament for use in combination with 3-(2-cyanophenyl)-5-(2-pyridyl)-1-phenyl-1,2-dihydropyridin-2-one, a pharmaceutically acceptable salt thereof, a hydrate thereof or a hydrate of a pharmaceutically acceptable salt thereof in the treatment of (i) Alzheimer's disease in a patient or (ii) dementia or one or more cognitive impairments in a patient.

7. The use of claim 5 or 6, wherein donepezil or the pharmaceutically acceptable salt thereof, and 3-(2-cyanophenyl)-5-(2-pyridyl)-1-phenyl-1,2-dihydropyridin-2-one, the pharmaceutically acceptable salt thereof, the hydrate thereof or the hydrate of a pharmaceutically acceptable salt thereof are (a) for administration separately to the patient or (b) for administration to the patient in the form of a pharmaceutical composition.

8. The use of any one of claims 5 to 7 wherein the dementia is caused by Alzheimer's disease, Parkinson's disease, or Huntington's disease.

## Patentansprüche

1. Verbindung zur Verwendung in Kombination mit Donepezil oder einem pharmazeutisch verträglichen Salz davon bei der Behandlung von (i) Alzheimer'scher Erkrankung bei einem Patienten oder (ii) Demenz oder einer oder mehrerer kognitiver Beeinträchtigungen bei einem Patienten, wobei die Verbindung 3-(2-Cyanophenyl)-5-(2-pyridyl)-1-phenyl-1,2-dihydropyridin-2-on, ein pharmazeutisch verträgliches Salz davon, ein Hydrat davon oder ein Hydrat eines pharmazeutisch verträglichen Salzes davon ist.

2. Verbindung zur Verwendung in Kombination mit 3-(2-Cyanophenyl)-5-(2-pyridyl)-1-phenyl-1,2-dihydropyridin-2-on, einem pharmazeutisch verträglichen Salz davon, einem Hydrat davon oder einem Hydrat eines pharmazeutisch verträglichen Salzes davon bei der Behandlung von (i) Alzheimer'scher Erkrankung bei einem Patienten oder (ii) Demenz oder einer oder mehrerer kognitiver Beeinträchtigungen bei einem Patienten, wobei die Verbindung Donepezil oder ein pharmazeutisch verträgliches Salz davon ist.

3. Verbindung gemäß Anspruch 1 oder 2, wobei Donepezil oder das pharmazeutisch verträgliche Salz davon und 3-(2-Cyanophenyl)-5-(2-pyridyl)-1-phenyl-1,2-dihydropyridin-2-on, das pharmazeutisch verträgliche Salz davon, das Hydrat davon oder das Hydrat eines pharmazeutisch verträglichen Salzes davon (a) getrennt an den Patienten verabreicht werden oder (b) dem Patienten in Form einer pharmazeutischen Zusammensetzung verabreicht werden.

4. Verbindung gemäß einem der vorangehenden Ansprüche, wobei die Demenz durch Alzheimer'sche Erkrankung, Parkinson'sche Erkrankung oder Huntington'sche Erkrankung verursacht wird.

5. Verwendung von 3-(2-Cyanophenyl)-5-(2-pyridyl)-1-phenyl-1,2-dihydropyridin-2-on, eines pharmazeutisch verträglichen Salzes davon, eines Hydrats davon oder eines Hydrats eines pharmazeutisch verträglichen Salzes davon bei der Herstellung eines Medikaments zur Verwendung in Kombination mit Donepezil oder einem pharmazeutisch verträglichen Salz davon bei der Behandlung von (i) Alzheimer'scher Erkrankung bei einem Patienten oder (ii) Demenz oder einer oder mehrerer kognitiver Beeinträchtigungen bei einem Patienten.

6. Verwendung von Donepezil oder einem pharmazeutisch verträglichen Salz davon bei der Herstellung eines Medikaments zur Verwendung in Kombination mit 3-(2-Cyanophenyl)-5-(2-pyridyl)-1-phenyl-1,2-dihydropyridin-2-on, einem pharmazeutisch verträglichen Salz davon, einem Hydrat davon oder einem Hydrat eines pharmazeutisch verträglichen Salzes davon bei der Behandlung von (i) Alzheimer'scher Erkrankung bei einem Patienten oder (ii) Demenz oder einer oder mehrerer kognitiver Beeinträchtigungen bei einem Patienten.

7. Verwendung gemäß Anspruch 5 oder 6, wobei Donepezil oder das pharmazeutisch verträgliche Salz davon und 3-(2-Cyanophenyl)-5-(2-pyridyl)-1-phenyl-1,2-dihydropyridin-2-on, das pharmazeutisch verträgliche Salz davon, das Hydrat davon oder das Hydrat eines pharmazeutisch verträglichen Salzes davon (a) zur getrennten Verabreichung an den Patienten oder (b) zur Verabreichung an den Patienten in Form einer pharmazeutischen Zusammensetzung sind.

8. Verwendung gemäß einem der Ansprüche 5 bis 7, wobei die Demenz durch Alzheimer'sche Erkrankung, Parkinson'sche Erkrankung oder Huntington'sche Erkrankung verursacht wird.

## Revendications

1. Composé pour utilisation en combinaison avec du donépézil ou un sel pharmacologiquement admissible de celui-ci dans le traitement
i) de la maladie d'Alzheimer chez un patient,
ii) ou d'une démence ou d'un ou de plusieurs trouble(s) cognitif(s) chez un patient,
lequel composé est la 3-(2-cyano-phényl)-5-(pyrid-2-yl)-1-phényl-1,2-dihydro-pyridine-2-one, un sel pharmacologiquement admissible de celle-ci, un hydrate de celle-ci ou un hydrate d'un sel pharmacologiquement admissible de celle-ci.

2. Composé pour utilisation en combinaison avec de la 3-(2-cyano-phényl)-5-(pyrid-2-yl)-1-phényl-1,2-dihydro-pyridine-2-one, un sel pharmacologiquement admissible de celle-ci, un hydrate de celle-ci ou un hydrate d'un sel pharmacologiquement admissible de celle-ci, dans le traitement
i) de la maladie d'Alzheimer chez un patient,
ii) ou d'une démence ou d'un ou de plusieurs trouble(s) cognitif(s) chez un patient,
lequel composé est du donépézil ou un sel pharmacologiquement admissible de celui-ci.

3. Composé conforme à la revendication 1 ou 2, le donépézil, ou son sel pharmacologiquement admissible, et la 3-(2-cyano-phényl)-5-(pyrid-2-yl)-1-phényl-1,2-dihydro-pyridine-2-one, ou son sel pharmacologiquement admissible, son hydrate ou l'hydrate de son sel pharmacologiquement admissible, étant
a) administrés séparément au patient,
b) ou administrés au patient sous la forme d'une composition pharmaceutique.

4. Composé conforme à l'une des revendications précédentes, la démence étant causée par la maladie d'Alzheimer, la maladie de Parkinson ou la chorée de Huntington.

5. Utilisation de la 3-(2-cyano-phényl)-5-(pyrid-2-yl)-1-phényl-1,2-dihydro-pyridine-2-one, d'un sel pharmacologiquement admissible de celle-ci, d'un hydrate de celle-ci ou d'un hydrate d'un sel pharmacologiquement admissible de celle-ci, dans la fabrication d'un médicament conçu pour être utilisé en combinaison avec du donépézil ou un sel pharmacologiquement admissible de celui-ci dans le traitement
i) de la maladie d'Alzheimer chez un patient,
ii) ou d'une démence ou d'un ou de plusieurs trouble(s) cognitif(s) chez un patient.

6. Utilisation du donépézil ou d'un sel pharmacologiquement admissible de celui-ci dans la fabrication d'un médicament conçu pour être utilisé en combinaison avec de la 3-(2-cyano-phényl)-5-(pyrid-2-yl)-1-phényl-1,2-dihydro-pyridine-2-one, un sel pharmacologiquement admissible de celle-ci, un hydrate de celle-ci ou un hydrate d'un sel pharmacologiquement admissible de celle-ci, dans le traitement
i) de la maladie d'Alzheimer chez un patient,
ii) ou d'une démence ou d'un ou de plusieurs trouble(s) cognitif(s) chez un patient.

7. Utilisation conforme à la revendication 5 ou 6, le donépézil, ou son sel pharmacologiquement admissible, et la 3-(2-cyano-phényl)-5-(pyrid-2-yl)-1-phényl-1,2-dihydro-pyridine-2-one, ou son sel pharmacologiquement admissible, son hydrate ou l'hydrate de son sel pharmacologiquement admissible, devant être
a) administrés séparément au patient,
b) ou administrés au patient sous la forme d'une composition pharmaceutique.

8. Utilisation conforme à l'une des revendications 5 à 7, la démence étant causée par la maladie d'Alzheimer, la maladie de Parkinson ou la chorée de Huntington.
